# EUROPÄISCHE PATENTSCHRIFT

(11) **EP 0 635 022 B1**
(45) Veröffentlichungstag und Bekanntmachung des Hinweises auf die Patenterteilung: **06.11.1996**
(21) Anmeldenummer: 93908869.6
(22) Anmeldetag: 01.04.1993
(51) Int. Cl.: C07H 15/04

(54) **VERFAHREN ZUR HERSTELLUNG VON ALKYLGLYKOSIDEN**
PROCESS FOR PRODUCING ALKYL GLYCOSIDES
PROCEDE DE PRODUCTION D'ALKYLGLUCOSIDES

(30) Priorität: 10.04.1992 DE 4212080
(43) Veröffentlichungstag der Anmeldung: 25.01.1995
(73) Patentinhaber: BASF Aktiengesellschaft, 67063 Ludwigshafen (DE)
(72) Erfinder: WOLF, Gerhard, D-6800 Mannheim 24 (DE); OFTRING, Alfred, D-6702 Bad Duerkheim (DE); SCHUH, Georg, D-6700 Ludwigshafen (DE); WOLF, Helmut, D-6733 Hassloch (DE); ALT, Rudolf, D-6700 Ludwigshafen (DE); BECHTOLSHEIMER, Hans-Heinrich, D-6521 Dittelsheim-Hessloch (DE); HERTEL, Dieter, D-6906 Leimen (DE)
(86) Internationale Anmeldenummer: EP9300791
(87) Internationale Veröffentlichungsnummer: WO9321196

(56) Entgegenhaltungen:
- EP-A- 0 132 043
- WO-A-90/07516

## Beschreibung

Die vorliegende Erfindung betrifft ein verbessertes Verfahren zur Herstellung von Alkylglykosiden durch Umsetzung von wäßrigen Glykosen mit einem Wassergehalt von 10 bis 80 Gew.-% mit aliphatischen primären Alkoholen mit 8 bis 30 C-Atomen.

Oberflächenaktive Alkylglykoside, die hauptsächlich auf dem Wasch- und Reinigungsmittelsektor Anwendung finden, sind seit langem bekannt und werden im technischen Maßstab nach zwei unterschiedlichen Verfahrenstypen hergestellt. Entweder wird nach der "Direktsynthese" die langkettige Alkoholkomponente mit der Zuckerkomponente unter Wasserabspaltung direkt verknüpft oder es wird nach der sogenannten Umacetalisierungsmethode zunächst als Zwischenprodukt ein kurzkettiges Alkylglykosid hergestellt, das dann in einer zweiten Stufe durch Umacetalisierung mit langkettigen Alkoholen zum oberflächenaktiven Alkylglykosid umgesetzt wird. Die Umacetalisierungsmethode weist allerdings gegenüber der Direktsynthese eine Reihe von prinzipiellen Nachteilen auf wie den zusätzlichen Einsatz eines kurzkettigen Alkohols, eine schlechtere Raum-Zeit-Ausbeute oder das Entstehen von relativ komplexen Produktgemischen.

Als Beispiel für die Umacetalisierungsmethode ist die EP-A 0 252 241 zu nennen. Hiernach werden Butyloligoglykoside durch säurekatalysierte Umsetzung, z.B. mit Schwefelsäure oder p-Toluolsulfonsäure, von wäßrigen Saccharidsirupen unter Zusatz von Butyloligosacchariden hergestellt. Die kurzkettigen Butylglykoside können dann einer Umacetalisierung zu längerkettigen Alkylglykosiden unterzogen werden.

In der EP-A 0 362 671 wird - wie auch in den nachfolgend zitierten Literaturstellen - eine Direktsynthese von längerkettigen Alkylglykosiden beschrieben. In dieser Schrift wird die Verwendung von sauren Katalysatoren wie Schwefelsäure, Phosphorsäure oder aliphatischen oder aromatischen Sulfonsäuren für die Acetalisierungsreaktion empfohlen. Die zur Umsetzung verwendeten Glykosen wie Glucose sollten möglichst wasserfrei sein. Man führt die Umsetzung beispielsweise so durch, daß man eine Suspension der Glykose in einem Fettalkohol kontinuierlich zu einer Mischung aus saurem Katalysator und Fettalkohol zugibt und dabei im Vakuum das entstehende Reaktionswasser abdestilliert.

In der EP-A 0 096 917 wird ein Verfahren beschrieben, bei dem man ein in einem Fettalkohol suspendiertes Monosaccharid wie Glucose kontinuierlich oder portionsweise so zu einer Mischung aus Fettalkohol und einem sauren Katalysator wie Schwefelsäure oder Toluolsulfonsäure bei 80 bis 150°C hinzufügt, daß nie mehr als 10 % nicht umgesetztes Monosaccharid im Reaktionsgemisch vorhanden sind. Es wird wasserfreie Glucose einer bestimmten Korngröße eingesetzt.

In der EP-B 0 132 043 wird für die Direktsynthese von Alkylglykosiden als saurer Katalysator die Säureform eines anionischen Tensides empfohlen, durch dessen Verwendung anstelle üblicher Katalysatoren, wie Schwefelsäure oder p-Toluolsulfonsäure, die Farbqualität des Produkts verbessert und der Gehalt des Endprodukts an unerwünschten Polysacchariden herabgesetzt wird soll. Bei dem Verfahren dieser Literaturstelle wird nur ein geringer Fettalkoholüberschuß, vorzugsweise 2 mol Fettalkohol pro Mol Glucose, die in ihrer wasserfreien Form verwendet wird, eingesetzt und bei der Neutralisierung des sauren Katalysators mit einer Base werden pH-Werte zwischen 6,6 und 7 eingestellt.

Auch gemäß WO-A-90/07516 und DE-A 39 27 919 wird durch Verwendung derartiger oberflächenaktiver saurer Katalysatoren - Dinonylnaphthalinsulfonsäure oder Sulfobernsteinsäure - ein Alkylglykosid mit einer helleren Farbe und einem geringen Gehalt an Polysacchariden erzielt.

Aus der US-A-4 721 780 ist ein Verfahren zur Herstellung von Alkylpolyglucosiden bekannt, bei dem man wäßrige Monosaccharidlösungen mit einwertigen C₂- bis C₆-aliphatischen Alkoholen in einer homogenen wäßrigen Phase in Gegenwart von sauren Katalysatoren bei Temperaturen von 60 bis 200°C umsetzt und Wasser aus dem Reaktionsgemisch in der Weise entfernt, daß sich keine separate Phase aus einer wäßrigen Monosaccharidlösung bildet.

Ein entsprechendes Verfahren ist aus der US-A-4 996 306 für die Herstellung von C₇- bis C₃₀-Alkylpolyglucosiden bekannt, bei dem man wäßrige Mono- oder Oligosaccharidlösungen mit C₇- bis C₃₀-Alkoholen in einem homogenen, einphasigen wäßrigen Reaktionsmedium umsetzt. Auch bei diesem Verfahren soll die Ausbildung einer getrennten Phase, die aus einer wäßrigen Saccharidenlösung besteht, vermieden werden. Die Beispiele der US-A-4 996 306 beziehen sich jedoch lediglich auf die säurekatalysierte Umsetzung von wäßrigen Saccharidlösungen mit n-Propanol, n-Butanol oder Gemischen von n-Butanol und Methanol, wobei Methanol zur Verbesserung der Mischbarkeit der Reaktionspartner eingesetzt wird, um die Reaktion in homogener Phase durchzuführen.

Die nach den aus dem Stand der Technik bekannten Verfahren hergestellten Alkylglykoside weisen allerdings immer noch eine Reihe von Nachteilen auf. Sie sind in der Regel immer noch zu stark gefärbt und weisen oft ein zu ungünstiges Hydrophil/Hydrophob-Verhältnis auf. Man ist nämlich bei Alkylglykosiden bestrebt, das Hydrophil/Hydrophob-Verhältnis zugunsten des hydrophoben Teils zu verschieben. Dies gelingt am besten durch eine gezielte Synthese von Alkylmonoglykosiden und Vermeidung der Alkyloligo- und -polyglykoside. Denn je höher der Anteil der Alkylmonoglykoside ist, desto hydrophober verhält sich naturgemäß das Produkt.

Außerdem wird eine weitere Vereinfachung des Herstellungsverfahrens für Alkylglykoside angestrebt. Das Herstellungsverfahren soll - auch im technischen Maßstab - hohe Effizienz haben und problemlos ausführbar sowie wirtschaftlich sein. Insbesondere sollen leicht zugängliche Einsatzstoffe verwendet werden können.

Aufgabe der vorliegenden Erfindung ist es, ein verbessertes Herstellungsverfahren für Alkylglykoside bereitzustellen, das Produkte mit verbesserten Eigenschaften liefert und effizient, problemlos ausführbar und wirtschaftlich ist.

Die Aufgabe wird erfindungsgmeäß gelöst mit einem zur Herstellung von Alkylglykosiden durch Umsetzung von wäßrigen Glykosen mit einem Wassergehalt von 10 bis 80 Gew.-% mit aliphatischen primären Alkoholen mit 8 bis 30 C-Atomen, wenn man
(a) die Alkohole und die Glykosen im molaren Verhältnis von 2:1 bis 10:1 einsetzt,
(b) als sauren Katalysator mit emulgierenden Eigenschaften 0,1 bis 5 Gew.-%, bezogen auf die Menge der eingesetzten Glykosen, der sauren Form eines anionischen Tensids verwendet,
(c) als weiteren Emulgator 1 bis 30 Gew.-%, bezogen auf die Menge der eingesetzten Glykosen, Alkylglykoside verwendet,
(d) die wäßrige Glykose auf 50 bis 90°C vorwärmt und die vorgewärmte wäßrige Lösung der Glykose unter effektiver Durchmischung zur Reaktionsmischung aus Alkohol, saurem Katalysator und Emulgator so zudosiert, daß eine Emulsion entsteht,
(e) die Umsetzung in Emulsion bei einer Temperatur von 100 bis 150°C und einem Druck von 10 bis 100 mbar vornimmt, wobei fortlaufend das in die Reaktionsmischung eingebrachte und das durch die Umsetzung entstehende Wasser abdestilliert wird,
(f) nach erfolgter Umsetzung den sauren Katalysator durch Zugabe einer Base neutralisiert, so daß die resultierende Mischung einen pH-Wert von 8 bis 10 aufweist, anschließend
(g) den überschüssigen Alkohol bei einem Druck von 0,01 bis 10 mbar bis auf einen Restgehalt von weniger als 5 Gew.-%, bezogen auf die Menge des vorliegenden Alkylglykosids, abdestilliert und
(h) das Reaktionsgemisch nach Überführen in eine wäßrige Paste mit einem Gehalt von 30 bis 70 Gew.-% Alkylglykosid bei pH 8 bis 10 mit einer Aktivsauerstoff abspaltenden Verbindung bleicht.

Unter Alkylglykosiden werden im folgenden die Reaktionsprodukte aus Zuckern und aliphatischen Alkoholen verstanden, wobei als Zuckerkomponente im folgenden als Glykosen bezeichnete Aldosen und Ketosen in Betracht kommen, z.B. Glucose, Fructose, Mannose, Galactose, Talose, Gulose, Allose, Altrose, Idose, Arabinose, Xylose, Lyxose oder Ribose. Vorzugsweise werden wegen der besseren Reaktionsfähigkeit die Aldosen verwendet. Unter den Aldosen kommt wegen ihrer leichten Zugänglichkeit und Verfügbarkeit in technischen Mengen insbesondere die Glucose in Betracht. Die nach dem erfindungsgemäßen Verfahren bevorzugt hergestellten Alkylglykoside sind deshalb die Alkylglucoside.

Der Begriff Alkyl in Alkylglykosid umfaßt den Rest eines primären aliphatischen Alkohols der Kettenlänge C8 bis C30, insbesondere eines aus natürlichen Fetten erhältlichen Fettalkohols. Die Begriffe Alkyloligoglykosid, Alkylpolyglykosid, Alkyloligosaccharid den Alkylpolysaccharid beziehen sich auf solche alkylierten Glykosen, in denen ein Alkylrest in Form des Acetals an mehr als einen Glykoserest, also an einen Poly- oder Oligosaccharidrest gebunden ist. Diese Begriffe werden als untereinander gleichbedeutend angesehen. Dementsprechend ist ein Alkylmonoglykosid das Acetal eines Monosaccharids. Da man bei der säurekatalysierten Umsetzung von Zuckern und aliphatischen Alkoholen im allgemeinen Gemische erhält, werden im folgenden unter dem Begriff Alkylglykosid sowohl Alkylmonoglykoside als auch Alkylpoly(oligo)glykoside und insbesondere Mischungen daraus - einschließlich eventueller Nebenkomponenten wie z.B.Fructosiden - verstanden.

Beim erfindungsgemäßen Verfahren wurde gefunden, daß bei der Synthese von langkettigen Alkylglykosiden nach der Direktfahrweise nicht mehr wasserfreie Glucose, die zum einen nur aufwendig herstellbar, zum anderen schlecht zu handhaben ist, oder Stärke, die vorher einer Abbau-Reaktion unterworfen werden muß und dafür Kosolvenzien benötigt, eingesetzt werden muß, sondern waßrige Glucose ("Dextrosesirup") verwendet werden kann. Eine derartige wäßrige Glucose-Lösung wird bei der enzymatischen oder sauren Hydrolyse von Stärke, z.B. Kartoffel-, Mais- oder Weizenstärke, erhalten und kann einen unterschiedlichen Gehalt an freier Glucose enthalten. So sollte der Anteil an freier, monomerer Glucose bei mindestens 50 Gew.-%, bezogen auf den Feststoffgehalt, liegen. Bevorzugt wird eine wäßrige Glucose-Lösung mit mindestens 80 Gew.-% monomerer Glucose, besonders bevorzugt ein Gehalt von mehr als 90 Gew.-% monomerer Glucose. Neben der monomeren Glucose können auch höhere oligomere und polymere Zuckerkomponenten vorliegen, je nachdem wie sie bei der Hydrolyse von Stärke entstehen.

Neben der wäßrigen Glucose können auch andere Glykosen, z.B. Fructose, Mannose, Glactose, Talose, Gulose, Allose, Altrose, Idose, Arabinose, Xylose, Lyxose und Ribose in ihrer wäßrigen Form mit den angegebenen Monomer-Gehalten eingesetzt werden. Auch Glykose-Gemische, Oligosaccharide, z.B. Maltose, Lactose, Multotriose oder Gemische aus Mono- und Oligosacchariden sind einsetzbar.

Der Wassergehalt bei den wäßrigen Glykosen liegt bei 10 bis 80 Gew.-%, vorzugsweise 15 bis 60 Gew.-%. Bevorzugt werden technische Lösungen, vor allem Dextrosesirup, die einen Wassergehalt von 20 bis 40 Gew.-% haben.

Der Vorteil des Einsatzes von wäßrigen Glykosen liegt nicht nur in der leichteren Verfügbarkeit der Glykoselösungen, sondern auch in dem leichteren "Handling", besonders in der leichteren Dosierbarkeit zur vorgewärmten Alkohol-Lösung.

Die eingesetzten aliphatischen primären Alkohole können praktisch beliebige Kettenlängen, d.h. solche von etwa 8 bis etwa 30 Kohlenstoffatomen, aufweisen. Um wirksame oberflächenaktive Reaktionsprodukte, die als Tensidrohstoffe in Wasch- und Reinigungsmitteln eingesetzt werden können, zu erhalten, werden aliphatische primäre Alkohole mit 8 bis 20 Kohlenstoffatomen, insbesondere solche mit 8 bis 18 Kohlenstoffatomen, bevorzugt. Diese höheren aliphatischen Alkohole werden vorzugsweise aus technischen Fetten hergestellt. Selbstverständlich ist es aber auch möglich, synthetische primäre Alkohole wie Oxoalkohole oder Ziegler-Alkohole beim erfindungsgemäßen Verfahren einzusetzen.

Bei den als Alkoholkomponente besonders wichtigen aliphatischen primären C₈- bis C₁₈-, insbesondere C₁₂- bis C₁₈-Alkoholen handelt es sich vorzugsweise um gesättigte und insbesondere um geradkettige Alkohole, wie sie durch die Hydrierung von nativen Fettsäuren im technischen Maßstab erhalten werden können. Denn ein wichtiger Aspekt beim erfindungsgemäßen Verfahren ist die Herstellung von Tensiden, die ausschließlich aus nachwachsenden Rohstoffen herstellbar sind. Typische Vertreter der höheren aliphatischen Alkohole, die beim erfindungsgemäßen Verfahren verwendet werden können, sind z.B. n-Octylalkohol, n-Nonylalkohol, n-Decylalkohol, n-Undecylalkohol, n-Dodecylalkohol, n-Tridecylalkohol, n-Tetradecylalkohol, n-Hexadecylalkohol oder n-Octadecylalkohol. Da die Fettalkohole bevorzugt aus natürlichen Fettquellen stammen, kommen üblicherweise auch Gemische technischer Fettalkohole als Reaktionspartner in Betracht.

Neben den eigentlichen Fettalkoholen sind auch verzweigtkettige primäre Alkohole wie 2-Ethylhexanol oder wie Oxoalkohole für die Umsetzung geeignet. Typische Oxoalkohole sind z.B. die Verbindungen C₁₂-C₁₃-Alkanol mit ca. 25 % hauptsächlich 2-Methylverzweigung (Dobanol 23) und das entsprechende C₉-C₁₁-Alkanol (Dobanol 91).

Die Ansatzverhältnisse werden so gewählt, daß das Molverhältnis aliphatischer Alkohol zu Glykose bei 2:1 bis 10:1, vorzugsweise 3:1 bis 8:1, insbesondere bei 3:1 bis 6:1 liegt (Maßnahme a).

Als saurer Katalysator mit emulgierenden Eigenschaften wird die saure Form von anionischen Tensiden eingesetzt (Maßnahme b). Als besonders geeignete Vertreter solcher Tenside sind zu nennen:
- Alkylbenzolsulfonsäuren, insbesondere solche der Formel 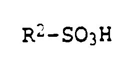 mit R² = C₈-C₃₀-Alkyl;
   mit R¹ = C₈-C₂₂-Alkyl (besonders bevorzugt sind Kettenlängen von C₁₂ bis C₁₄);
- Alkylsulfonsäuren, insbesondere solche der Formel

   R²-SO₃H mit R² = C₈-C₃₀-Alkyl;
- Sulfobernsteinsäuremono- oder -diester, insbesondere solche der Formel 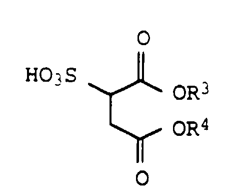 mit R³ = H oder C₆-C₂₂-Alkyl
   und R⁴ = C₆-C₂₂-Alkyl;
- Sulfoalkylcarbonsäuren oder -carbonsäureester, insbesondere solche der Formel 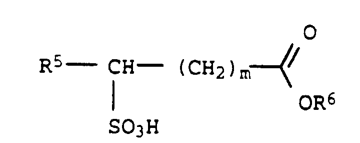 mit R⁵ = C₄-C₂₂-Alkyl,
   R⁶ = C₁-C₃₀-Alkyl oder H
   und m = 0 bis 10;
- Mono- und Dialkylnaphthalinsulfonsäuren, insbesondere solche der Formel 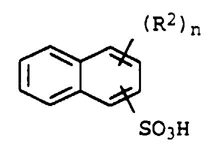 mit R² = C₈-C₃₀-Alkyl
   und n = 1 bis 2.
Wird bei dem erfindungsgemäßen Verfahren nicht ein saurer Katalysator eingesetzt, der gleichzeitig als Emulgator für die Glykose im Alkohol wirken kann, so ist das Verfahren nicht durchführbar. So treten beispielsweise mit p-Toluolsulfonsäure starke Verklumpungen auf und es kommt zur vermehrten Bildung von Polyglykosen. Besonders bevorzugt werden Alkylbenzolsulfonsäuren und hier insbesondere Benzolsulfonsäuren mit C₁₂- bis C₁₄-Alkylresten in p-Stellung. Die Katalysator-Konzentration liegt zwischen 0,1 und 5 Gew.-%, bezogen auf die Menge der eingesetzten Glykose. Bevorzugt wird eine Katalysator-Konzentration von 0,5 bis 2 Gew.-%.

Es wurde gefunden, daß bei Zusatz eines weiteren Emulgators zur Reaktionslösung zusätzlich zum emulgierend wirkenden sauren Katalysator eine deutliche Verbesserung des Verfahrens erreicht wird. Als weiterer Emulgator werden hierfür Alkylglykoside, vor allem Alkylglucosid eingesetzt (Maßnahme c).

Alkylglykoside können als Festsubstanzen oder wäßrige Lösungen eingesetzt werden. Vorteilhafterweise werden jedoch alkoholische Lösungen von Alkylglykosiden verwendet. Das erfindungsgemäße Verfahren sieht in einer bevorzugten Ausführungsform vor, daß man von dem erzeugten Alkylglykosid, das nach Abschluß der Glykosidierung und Neutralisation im Alkohol gelöst vorliegt, eine Teilmenge zurückbehält und diese bei einer folgenden Charge wieder einsetzt. Dadurch braucht der Emulgator nicht isoliert zu werden. Diese Vorgehensweise hat den Vorteil, daß bei Beginn der Glykose-Dosierung sofort durch das im Reaktionsgefäß vorliegende Alkylglykosid/Alkohol-Gemisch eine Emulgierung der Glykose bewirkt wird. Dadurch kann die Glykose zu Beginn der Dosier-Phase besser abreagieren und es kommt zu keinen Eintrübungen und Ausfällungen, die auf eine vermehrte Bildung von Polyglykosen zurückzuführen sind. Wird der Emulgator weggelassen, tritt starke Polyglykose-Bildung auf, d.h. der zusätzliche Emulgator ist unverzichtbar.

Als zusätzlicher Emulgator können alle Alkylglykoside, die nach dem erfindungsgemäßen Verfahren hergestellt werden können, eingesetzt werden. Die Emulgatoren sind damit nicht auf Glucose-Derivate beschränkt. Als Emulgatoren können sowohl reine Alkylmonoglykoside, als auch technische Gemische aus Alkylmonoglykosiden, Alkyloligoglykosiden und Polyglykosen eingesetzt werden. Dies beeinträchtigt nicht deren Wirksamkeit.

Vorteilhafterwiese wird, wie oben beschrieben, eine alkoholische Lösung eines technischen Alkylglykosid-Gemenges verwendet. Als Berechnungsgrundlage für die eingesetzte Emulgator-Menge bei diesen alkoholischen Lösungen werden alle Zuckerkomponenten (Alkylmonoglykoside, Alkyloligoglykoside, Polyglykose) zusammengefaßt und auf die eingesetzten Glykose bezogen. Es können Emulgator-Konzentrationen zwischen 1 und 30 Gew.-%, vorzugsweise 1 bis 15 Gew.-% eingesetzt werden. Besonders bevorzugt werden Konzentrationen zwischen 1,0 bis 8 Gew.-%.

Bei der Durchführung des erfindungsgemäßen Verfahrens geht man so vor, daß man die wäßrige Glykose auf 50 bis 90°C, besser 60 bis 70°C vorwärmt (Maßnahme d). Dies führt zu einer deutlichen Viskositätserniedrigung, so daß die Glykose als leicht fließfähige Flüssigkeit dosiert werden kann. Zudem ist eine geringe Temperaturdifferenz zwischen zudosierter wäßriger Glykose-Lösung und dem Reaktionsgemisch wünschenswert, um die Zufuhr der Wärmeenergie bei der Reaktion zu erleichtern.

Die Zudosierung der waßrigen Glykolselösung wird portionsweise oder besser kontinuierlich durchgeführt. Die kontinuierliche Zufuhr wird vorteilhafterweise so gesteuert, daß gleichzeitig das eingetragene Wasser und das aus der Reaktion entstandene Wasser unter Vakuum abdestilliert wird. Dabei wird die Reaktion in heterogener Phase unter Bildung einer stabilen Glucosesirup/Alkohol-Emulsion durchgeführt, eine besondere Vorsicht bei der Dosierung zwecks ständiger Bildung einer homogenen Phase - wie in der US-A-4 996 306 beschrieben - braucht nicht eingehalten zu werden. Es muß lediglich eine gute Emulgierung des zudosierten Glykosesirups gewährleistet sein. Dies kann in einfacher Weise mit den schon beschriebenen emulgierenden Zusätzen (emulgierende Säure, Alkylglykosid als Emulgator) erreicht werden. Eine weitere effektive Maßnahme zur guten Emulgierung wird durch eine effektive Rührung bzw. Durchmischung der Reaktionsmischung erreicht, die im Labormaßstab vorzugsweise durch Strombrecher und Scheibenrührer bewirkt wird.

Bei Technikums- und Betriebsansätzen haben sich besonders Umpump-Einrichtungen über einen externen Flüssigkeitskreislauf bewährt, die zudem den Vorteil haben, daß über einen Wärmetauscher die Wärmeenergie schonend zugeführt werden kann und damit hohe Kesselwand-Temperaturen vermieden werden. Somit lassen sich negative Auswirkungen der Temperaturführung auf die Farbe des Produktes verhindern.

Besonders vorteilhaft hat sich eine Einspeisung bzw. Zudosierung des Glykosesirups in die Umpumpleitung via Flüssigkeitsstrahlpumpe erwiesen. Durch entsprechende Wahl der Unpumpleistungen (Treibstrahl) und der Dosiergeschwindigkeit läßt sich eine optimale Emulgierung erreichen, so daß im Reaktor nahezu keine Ausfällungen an Polyglykose festzustellen sind.

Erstaunlicherweise konnte festgestellt werden, daß bei der beschriebenen heterogenen Direktfahrweise unter Bildung einer Emulsion die Produktqualität bezüglich z.B. Monoglucosid-Gehalt, Polyglykose, Farbe besser als bei Literatur-bekannten Umacetalisierungsmethoden ist (vgl. Beispiel D). Sogar geringe partikelartige Ausfällungen an polymeren Produkten (überwiegend Polyglykose) beeinträchtigen die Produktqualität nicht. Allerdings müssen die partikelartigen Ausfällungen unter 2 Gew.-% bezüglich isoliertem Produkt liegen, da sonst zum einen Anbrennungen und Farbschädigungen des Produktes bei der Destillation auftreten, zum anderen die Ausbeuten an Alkylmonoglucosid geringer sind (siehe Vergleichsbeispiele A, B und C).

Prinzipiell können natürlich durch Filtration des Rohproduktes derartige partikelartige Ausfällungen an Polyglykose beseitigt werden (vgl. Vergleichsbeispiel A), was jedoch einen zusätzlichen Verfahrensschritt bedeutet. Bei dem obigen Verfahren ist dies jedoch bei geringen Ausfällungen (< 2 Gew.-% bzgl. isoliertem Produkt) nicht nötig.

Da bei dem vorliegenden Verfahren die Produktqualität weder durch das Arbeiten in heterogener Phase unter Bildung einer stabilen Emulsion, noch durch die Bildung geringer Ausfällungen beeinträchtigt wird, stellt das Verfahren einen vereinfachten, wirtschaftlichen Weg zur Synthese von Alkylpolyglykosiden und damit eine Verbesserung des Standes der Technik dar.

Die Reaktionsmischung aus Alkohol, saurem Katalysator und Alkylglykosiden wird vor Beginn der Glykose-Dosierung auf 100 bis 150°C, besser 110 bis 120°C vorgewärmt und die Umsetzung wird in diesem Temperaturbereich vorgenommen (Maßnahme e). Das mit der Glykose-Dosierung zugeführte Wasser und das bei der Acetal-Bildung entstehende Wasser wird unter Vakuum fortlaufend aus der Emulsion abdestilliert (Maßnahme e). Der Druck wird je nach eingesetztem höheren Alkohol zwischen 10 und 100 mbar gewählt. Je höher der Siedepunkt des eingesetzten Alkohols oder der Alkohol-Gemische ist, desto niedriger kann das Vakuum gewählt werden. So wird z.B. bei einem geradkettigen C₈/C₁₀-Alkohol-Gemisch ein Vakuum von zweckmäßigerweise 50 bis 60 mbar gewählt, während bei einem C₁₂/C₁₄-Alkohol-Gemisch ein Vakuum von 30 bis 40 mbar bevorzugt wird.

Nach Abschluß der Glykose-Dosierung wird zweckmäßigerweise noch 10 min bis 2 h im angegebenen Temperaturbereich nachgerührt. Danach ist der Gehalt an freier Glykose in der Reaktionslösung normalerweise auf unter 0,5 Gew.-% gesunken, d.h. die Glykose hat praktisch vollständig abreagiert.

Als Neutralisationsmittel zur Desaktivierung des sauren Katalysators nach der Umsetzung (Maßnahme f) eignen sich vor allem basische Alkalimetall-, Erdalkalimetall- oder Aluminiumsalze, deren Anionen organischer oder anorganischer Natur sein können. Beispiele derartiger Basen sind Natriumhydroxid, Kaliumhydroxid, Calciumhydroxid, Aluminiumhydroxid, Natriumacetat, Natriummethylat und Natriumethylat. Bevorzugt wird der Einsatz von wäßriger Natronlauge, da diese sich gut in die Alkohol/Produkt-Mischung einarbeiten läßt, gut dosierbar ist und keinen Filtrationsschritt für eventuell ausfallende Salze benötigt. Die Neutralisation wird so durchgeführt, daß anschließend eine leicht basische Lösung mit pH 8 bis 10, gemessen als 1:1-Gemisch von Wasser:Fettalkohol/Produkt, vorliegt. Zur Neutralisation kühlt man die heiße Reaktionslösung zweckmäßigerweise vorher etwas ab, etwa auf 70 bis 100°C.

Die Destillation des überschüssigen Alkohols (Maßnahme g) erfolgt mit bekannten, das Verfahrensprodukt schonenden Techniken, z.B. mittels eines Dünnschichtverdampfers, und wird bei Drücken von 0,01 bis 10 mbar durchgeführt, wobei der Druck vom Siedepunkt des eingesetzten Alkohols oder Alkohol-Gemisches abhängig ist. Hierzu ist eine vorherige Filtration von beispielsweise ausgefallener Polyglykose in der Regel nicht notwendig.

Das erhaltene, nahezu alkoholfreie Produkt wird durch Zugabe von Wasser zu einer 30 bis 70 gew.-%igen, vorzugsweise ca. 50 gew.-%igen wäßrigen Paste verarbeitet und mit einer Aktivsauerstoff abspaltenden Verbindung wie beispielsweise 30 gew.-%iger wäßriger Wasserstoffperoxid-Lösung gebleicht (Maßnahme h). Die Bleichung wird üblicherweise bei 70 bis 90°C durchgeführt, wobei der pH-Wert während des Bleichvorganges kontrolliert wird und gegebenenfalls mit beispielsweise Natriumhydroxid auf Werte zwischen pH 8 und pH 10 reguliert wird. Die verwendete Wasserstoffperoxid-Menge liegt in der Regel zwischen 0,3 und 3 Gew.-%, berechnet als H₂O₂ und bezogen auf die Menge des Produktes nach der Alkohol abtrennung.

Die vorliegende Erfindung stellt eine Kombination der Einzelmaßnahmen (a) bis (h) dar, wobei die Einzelmaßnahmen zum Teil als solche schon aus dem Stand der Technik bekannt waren. Die Durchführung der Glykosidierung in zwei Phasen, die als Emulsion vorliegen und die praktisch keine Polyglykose oder weniger als 2 Gew.-% Polyglykose enthalten, ist neu. Der Vorzug des erfindungsgemäßen Verfahrens liegt in der Kombination von mehreren aus den Einzelmaßnahmen (a) bis (h) herrührenden Einzelvorteilen, deren Zusammenwirken zu einem hervorragenden und überraschenden Gesamtergebnis führt.

Mit dem erfindungsgemäßen Verfahren erhält man insbesondere eine hervorragende Produktqualität, die hergestellten Alkylglykoside sind nur noch schwach gefärbt und weisen einen nur geringen Anteil an Alkyloligo- und -polyglykosiden auf.

Man kann in vorteilhafter Weise leicht verfügbare und gut handhabbare wäßrige Glykosid-Lösungen als Ausgangsmaterial verwenden und braucht nicht mehr auf durch umständliche Verfahren entwässerte und gegebenenfalls auf bestimmte Korngrößen verarbeitete feste Glykoside zurückzugreifen.

Das erfindungsgemaße Verfahren ist leicht und problemlos ausführbar, es gibt beispielsweise keine Dosierprobleme oder Schwierigkeiten bei der Wärmezu- oder -abfuhr, auch sind normalerweise keine umständlichen Filtrationsvorgänge notwendig. Das Verfahren ist einfach, es kommt mit einem Minimum an chemischen Reaktionspartnern und einem Minimum an Verfahrensmaßnahmen aus. Das Verfahren arbeitet mit hoher Effizienz bezüglich Raum-Zeit-Ausbeute, die erhaltenen Produkte sind ausreichend rein. Somit ist das Verfahren in hohem Maße wirtschaftlich.

Das erfindungsgemäße Verfahren gestattet schließlich eine problemlose Übertragung in den großtechnischen Maßstab ohne Scaling-up-Probleme.

### Beispiele

Die angegebenen Prozentangaben beziehen sich auf das Gewicht.

### Beispiel 1

In einem 2-l-Mehrhalsrührreaktor mit Strombecher, Scheibenrührer, Thermometer, Destillationsaufsatz und einer Dosiereinheit, bestehend aus einer Dosierpumpe, Druckhalteventil und einer Düse, wurden 825 g Dodecanol (4,57 mol) vorgelegt und dazu 2,6 g (0,008 mol) Dodecylbenzolsulfonsäure gegeben. Diese Mischung wurde mit 121,6 g eines Alkylglucosid/Dodekanol-Emulgator-Gemenges (Zusammensetzung: 63 % Dodecanol (0,41 mol), 22,6 % C₁₂-Monoglucosid, 5,2 % C₁₂-Diglucosid, 2,2 % C₁₂-Triglucosid, 0,7 % C₁₂-Tetraglucosid, < 0,5 % C₁₂-Pentaglucosid, 5,9 % Polyglucose) versetzt. Hierbei betrugen die Glucose-enthaltenden Anteile des Emulgators 30 % bezüglich der eingesetzten Glucose. Das molare Verhältnis von Fettalkohol zu Glucose betrug 6:1.

Die Lösung wurde auf 115 bis 120°C erhitzt. Bei einem Vakuum von 30 bis 35 mbar wurden kontinuierlich 214 g (0,83 mol) eines auf 60°C erwärmten Dextrosesirups (70 %ige Lösung, Glukose-Gehalt ca. 99,5 %) so zudosiert, daß eine trüb erscheinende Emulsion entsteht, die praktisch keine teilchenförmigen Ausfällungen an Polyglucose enthält. Gleichzeitig wurde bei dem angegebenen Vakuum sowohl das mit dem Sirup zugeführte Wasser als auch das bei der Reaktion erzeugte Wasser dem Gleichgewicht durch Destillation entzogen. Wichtig war hierbei, daß durch die Kombination Scheibenrührer (200 U/min), Strombrecher und Dosierung über eine Düse eine optimale Verteilung des Dextrosesirups im Fettalkohol gewährleistet wurde. Nach 4 h Dosierzeit und 30 min Nachrührzeit waren 83 g Wasser abdestilliert. Man erhielt eine leicht trübe, hellgelbe Reaktionslösung.

Nach Abkühlen auf 90°C wurde mit 1,6 g 50 %iger Natronlauge der Katalysator desaktiviert, die resultierende Lösung hatte einen pH-Wert von 8,3 (gemessen in 50 %iger wäßriger Lösung). Der überschüssige Alkohol wurde mittels Dünnschichtverdampfer (Heiztemperatur 170°C, Ablauftemperatur 140°C) bei einem Vakuum von 1 mbar entfernt. Die Destillatmenge betrug 820 g. Das Produkt (240 g) wurde durch Wasserzugabe direkt zu einer 50 %igen waßrigen Lösung in Pastenform verarbeitet und mit 12,3 g H₂O₂ (30 %ige Lösung) bei 80°C gebleicht. Die Iodfarbzahl betrug 15. Die Zusammensetzung des Produktes ist in der unten angeführten Tabelle wiedergegeben.

### Vergleichsbeispiel A (p-Toluolsulfonsäure als Katalysator)

In Beispiel 1 wurde die Dodecylbenzolsulfonsäure durch 1,5 g (0,008 mol) p-Toluolsulfonsäure ersetzt. Man erhielt bei zu Beispiel 1 analoger Fahrweise nach der Dosierung des Dextrosesirups ein stark trübes, mit Feststoff-Partikeln durchsetztes Rohprodukt. Eine Filtration ergab 25 g Rückstand, der zu ca. 80 % aus Polyglucose bestand. Das analog Beispiel 1 aufgearbeitete Filtrat (210 g) hatte die in der unten aufgeführten Tabelle wiedergegebene Zusammensetzung.

### Vergleichsbeispiel B (schlechte Dispergierung)

Beispiel 1 wurde wiederholt, nur im Gegensatz zu Beispiel 1 wurde ein Tropftrichter statt der Düse zur Dosierung des Dextrosesirups eingesetzt. Nach der Dosierung des Sirups erhielt man eine trübe Lösung, die starke Verklumpungen aufwies. Eine Filtration ergab 64 g Rückstand (ca. 80 % Polyglukose).

Der Verzicht auf Strombrecher und Scheibenrührer führte zu einer noch schlechteren Dispergierung des Dextrosesirups. Es wurden nach Filtration über 50 % der Glucose als Polyglucose isoliert.

### Beispiele 2 bis 5

Es wurde unter Variation der Alkylglucosid-Konzentration wie in Beispiel 1 verfahren. Die Ergebnisse sind in der unten aufgeführten Tabelle zusammengefaßt.

### Vergleichsbeispiel C (ohne Alkylglucosid als Emulgator)

Beispiel 1 wurde wiederholt, nur im Gegensatz zu Beispiel 1 wurde kein Alkylglucosid als zusätzlicher Emulgator zugegeben. Nach der Dosierung des Dextrosesirups lag eine trübe, mit Feststoffpartikeln durchsetzte Lösung vor. Durch Filtration wurden 47 g Rückstand, der zu über 80 % aus Polyglucose bestand, abgetrennt. Das analog Beispiel 1 aufgearbeitete Filtrat (195 g) hatte die in der unten aufgeführten Tabelle wiedergegeben Zusammensetzung.

### Beispiel 6

Entsprechend Beispiel 4 wurden 606 g Dodekanol (3,25 mol), 16,7 g des gleichen Alkylglucosid/Dodekanol Emulgator-Gemenges wie in Beispiel 1 (3 % bez. auf eingesetzte Glucose) und 2,6 g (0,008 mol) Dodecylbenzolsulfonsäure mit 214 g (0,83 mol) des gleichen Dextrosesirups wie in Beispiel 1 unter Bildung einer Emulsion umgesetzt. Das molare Verhältnis von Fettalkohol zu Glucose betrug 4:1. Nach der Wasserabtrennung wurde eine leicht trübe Lösung erhalten. Die Destillation ergab 500 g Destillat und 245 g Produkt, das nach Bleichung mit 30 %iger H₂O₂-Lösung (12,3 g) eine Iodfarbzahl von 13 hatte (als 50 % wäßrige Paste). Die Zusammensetzung des Produktes ist in der unten aufgeführten Tabelle wiedergegeben.

### Beispiele 7 und 8

Es wurde unter weiterer Variation des Molverhältnisses Fettalkohol zu Glucose wie in Beispiel 6 verfahren. Die Ergebnisse sind in der unten aufgeführten Tabelle zusammengefaßt. Die Ergebnisse zeigen, daß mit abnehmenden Molverhältnissen der Alkylmonoglucosid-Gehalt sinkt und der Polyglucose-Gehalt steigt. Ein akzeptables Verhältnis zwischen Produkt-Zusammensetzung und Auslastung des Kolben- bzw. Kessel-Volumens ist bei Molverhältnissen von 3:1 bis 4:1 erreicht.

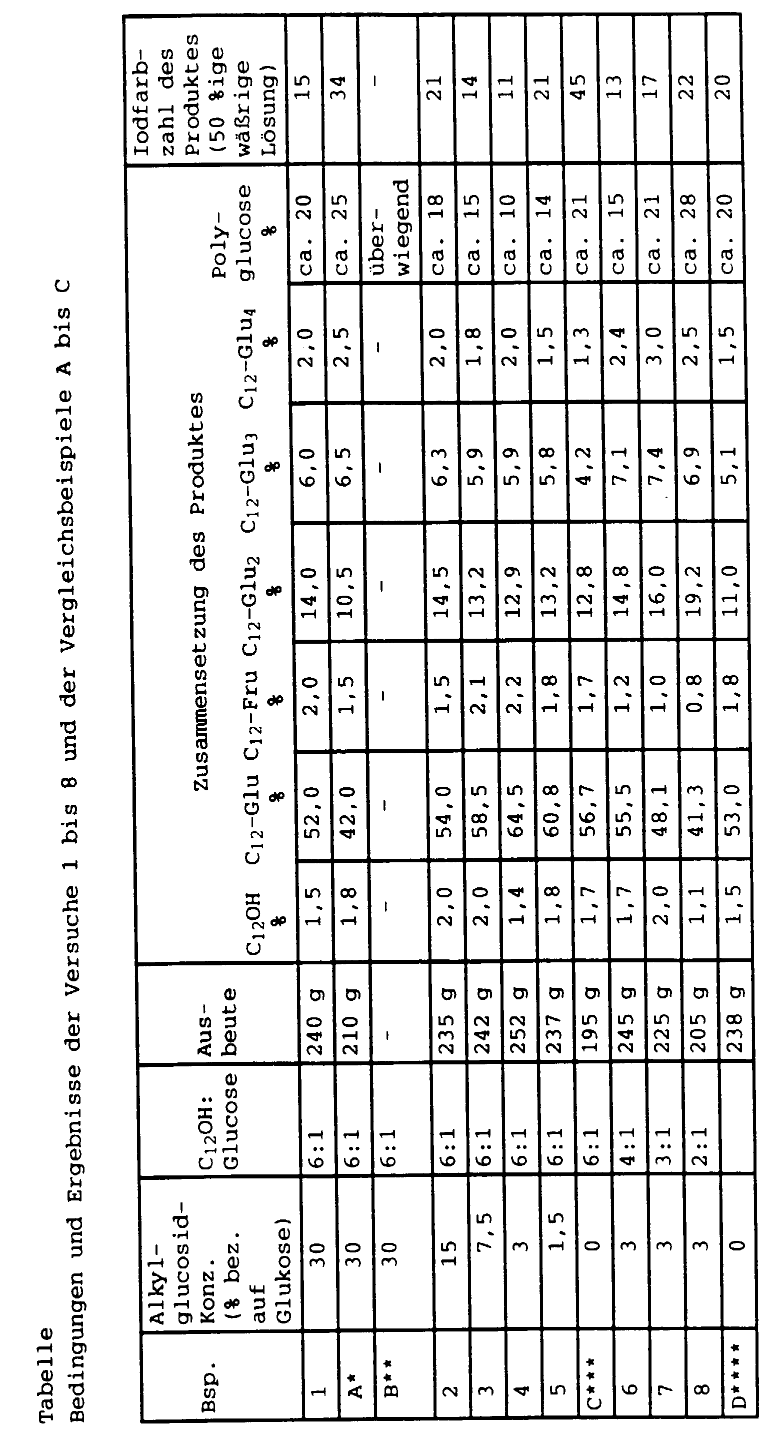

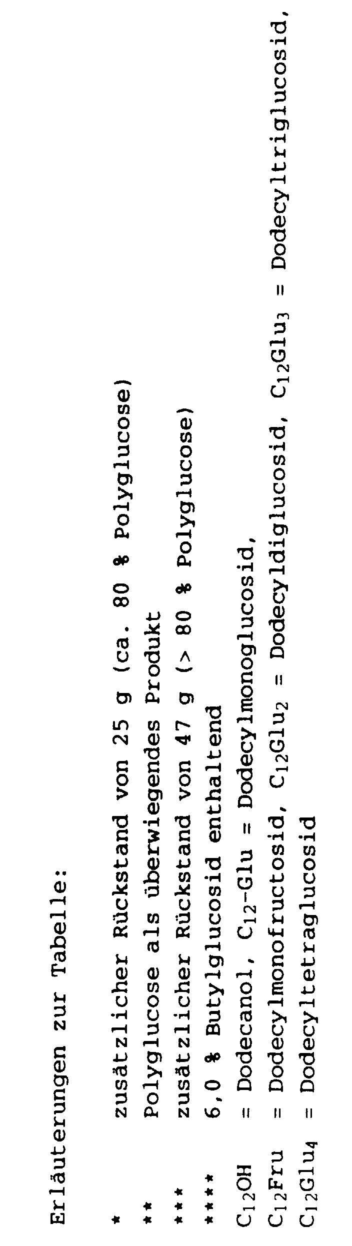

### Beispiel 9

Entsprechend Beispiel 6 wurden als Alkohol Komponenten 630 g Lorol 1214 spezial (Fettalkohol-Gemisch der Firma Henkel mit ca. 75 % Dodekanol, ca. 23 % Tetradekanol und ca. 1 % Hexadekanol; OHZ = 295 mg KOH/g) eingesetzt. Die Emulgator-Konzentration betrug 3 %, bezogen auf eingesetzte Glucose. Die Zusammensetzung des Emulgators betrug 64 % C₁₂/C₁₄-Alkohol, 23 % Alkylmonoglukosid, ca. 5 % Alkyldiglucosid, ca. 2 % Alkyltriglucosid, < 0,5 % Alkyltetra- und Alkylpentaglucosid, ca. 5 % Polyglucoside (Alkyl: C₁₂/C₁₄/C₁₆-Gemisch). Nach der Dosierung von Dextrosesirup liegt das Reaktionsgemisch als Emulsion vor. Daraus erhält man nach der Wasserabtrennung eine leicht trübe Lösung. Nach destillativer Aufarbeitung und Bleichung mit 30 %iger H₂O₂-Lösung (14,7 g) erhielt man 510 g Produkt als 50 %ige waßrige Lösung mit einer Iodfarbzahl von 9. Die Zusammensetzung des Produktes betrug 1,9 % C₁₂/C₁₄-Alkohol; 57 % Dodecyl/Tetradecylmonoglucosid, 1,9 % Dodecyl/Tetradecylfructosid, 14,4 % Dodecyl/Tetradecyldiglucosid, 7,7 % Dodecyl/Tetradecyltriglucosid, < 3 % Dodecyl/Tetradecyltetra- und Pentaglucosid, ca. 15 % Polyglucose, freie Glucose < 0,5 %. Die Hexadecyl-Anteile wurden nicht analysiert.

Andere native Alkoholgemische wie C₈/C₁₀- oder C₁₀-/C₁₂-Alkoholgemische (z.B. Lorol C 8-10 oder Lorol C 10-12 der Firma Henkel) ergaben ein analoges Spektrum in der Produkt-Zusammensetzung.

### Vergleichsbeispiel D

Analog dem aus der EP-A-0 301 298 bekannten Stand der Technik wurde nach der Umacetalisierungsmethode über die Butanolglucosid-Zwischenstufe ein Alkylpolyglucosid auf Basis Dodekanol hergestellt. Die Molverhältnisse Butanol:Glucose:Dodekanol betrugen 6:1:6. Die Zusammensetzungen des Produktes ist in der Tabelle zu entnehmen und zeigt deutlich, daß mit der beschriebenen Direktfahrweise ein qualitativ besseres Produkt hergestellt werden kann.

### Beispiel 10

zur Anwendung kam ein 335 l Email-Kessel mit Impeller-Rührer und einer externen Umpumpeinrichtung bestehend aus Umpumpleitung und Förderpumpe. In die Umpumpleitung war eine Flüssigkeitstrahlpumpe (Firma Wiegand) zur Dosierung des Glucosesirups eingebaut.

Folgende Stoffmengen wurden eingesetzt:
- 150 kg: Lorol 1214 spezial (vgl. Beispiel 9)
- 4,2 kg: Emulgator-Gemisch (Zusammensetzung analog Bsp. 9)
- 0,6 kg: Dodecylbenzolsulfonsäure
- 51,5 kg: Glucosesirup (70 %ige waßrige Lösung)

In dem Reaktor wurden Lorol 1214 spezial, Emulgator-Gemisch und Katalysator vorgelegt, auf 115 bis 120°C erwärmt und mit einer Umpumpleistung von 600 l/h durchmischt. Innerhalb von 4 h wurde auf 60°C vorgewärmter Glucosesirup über die Flüssigkeitsstrahlpumpe zudosiert und gleichzeitig Lösungsmittel- und Reaktionswasser bei einem Druck von 40 bis 50 mbar abdestilliert. Die abgetrennte Wassermenge betrug 20,2 kg. Es wurde nach Ende der Sirup-Dosierung 30 min nachgerührt, wobei nahezu keine Partikel-Ausfallungen an Polyglukose festzustellen waren (< 1 % bezogen auf Endprodukt). Nachfolgend wurde mit 200 g 50 %iger waßriger Natronlauge neutralisiert. Nach destillativer Aufarbeitung und Bleichung des Produktes mit 30 %iger H₂O₂-Lösung (0,8 kg) erhielt man 115 kg Produkt als 50 %ige waßrige Lösung (Jodfarbzahl 7). Die Zusammensetzung des Produktes betrug 1,6 % Restalkohol, 56,5 % Dodecyl/Tetradecylmonoglucosid, 2,1 % Dodecyl/Tetradecylfructosid, 13,3 % Dodecyl/Tetradecyldiglucosid, 7,5 % Dodecyl/Tetradecyltriglucosid, < 3 % Dodecyl/Tetradecyltetra- und pentaglucosid, ca. 16 % Polyglucose, < 0,5 % freie Glucose. Die Hexadecyl-Anteile wurden nicht analysiert.

## Patentansprüche

1. Verfahren zur Herstellung von Alkylglykosiden durch Umsetzung von wäßrigen Glykosen mit einem Wassergehalt von 10 bis 80 Gew.-% mit aliphatischen primären Alkoholen mit 8 bis 30 C-Atomen, dadurch gekennzeichnet, daß man
(a) die Alkohole und die Glykosen im molaren Verhältnis von 2:1 bis 10:1 einsetzt,
(b) als sauren Katalysator mit emulgierenden Eigenschaften 0,1 bis 5 Gew.-%, bezogen auf die Menge der eingesetzten Glykosen, der sauren Form eines anionischen Tensids verwendet,
(c) als weiteren Emulgator 1 bis 30 Gew.-%, bezogen auf die Menge der eingesetzten Glykosen, Alkylglykoside verwendet,
(d) die wäßrige Glykose auf 50 bis 90°C vorwärmt und die vorgewärmte waßrige Lösung der Glykose unter effektiver Durchmischung zur Reaktionsmischung aus Alkohol, saurem Katalysator und Emulgator so zudosiert, daß eine Emulsion entsteht,
(e) die Umsetzung in Emulsion bei einer Temperatur von 100 bis 150°C und einem Druck von 10 bis 100 mbar vornimmt, wobei fortlaufend das in die Reaktionsmischung eingebrachte und das durch die Umsetzung entstehende Wasser abdestilliert wird,
(f) nach erfolgter Umsetzung den sauren Katalysator durch Zugabe einer Base neutralisiert, so daß die resultierende Mischung einen pH-Wert von 8 bis 10 aufweist, anschließend
(g) den überschüssigen Alkohol bei einem Druck von 0,01 bis 10 mbar bis auf einen Restgehalt von weniger als 5 Gew.-%, bezogen auf die Menge des vorliegenden Alkylglykosids, abdestilliert und
(h) das Reaktionsgemisch nach Überführen in eine waßrige Paste mit einem Gehalt von 30 bis 70 Gew.-% Alkylglykosid bei pH 8 bis 10 mit einer Aktivsauerstoff abspaltenden Verbindung bleicht.

2. Verfahren zur Herstellung von Alkylglykosiden nach Anspruch 1, dadurch gekennzeichnet, daß man als wäßrige Glykose eine Lösung, die hauptsächlich Glukose als Glykose enthält, einsetzt.

3. Verfahren zur Herstellung von Alkylglykosiden nach Anspruch 1 oder 2, dadurch gekennzeichnet, daß man als Alkohole aliphatische primäre Alkohole mit 8 bis 20 C-Atomen einsetzt.

4. Verfahren zur Herstellung von Alkylglykosiden einem der den Ansprüche 1 bis 3, dadurch gekennzeichnet, daß man als saure Katalysatoren Alkylbenzolsulfonsäuren, Alkylsulfonsäuren, Sulfobernsteinsäuremono- und -diester, Sulfoalkylcarbonsäuren und -carbonsäureester sowie Mono- und Dialkylnaphthalinsulfonsäuren verwendet.

## Claims

1. A process for preparing alkyl glycosides by reaction of aqueous glycoses having a water content of 10 - 80% by weight with aliphatic primary alcohols having from 8 to 30 C atoms, which comprises
(a) employing the alcohols and the glycoses in a molar ratio of 2:1 - 10:1,
(b) using 0.1 - 5% by weight, based on the amount of the glycoses employed, of the acidic form of an anionic surfactant as the acidic catalyst having emulsifying properties,
(c) using 1 - 30% by weight, based on the amount of the glycoses employed, of alkyl glycosides as a further emulsifier,
(d) preheating the aqueous glycose to 50 - 90°C and metering the preheated aqueous solution of the glycose with effective mixing into the reaction mixture of alcohol, acidic catalyst and emulsifier in such a manner that an emulsion is formed,
(e) carrying out the reaction in emulsion at from 100 to 150°C and from 10 to 100 mbar, the water introduced into the reaction mixture and the water formed by the reaction being continuously removed by distillation,
(f) after the reaction has ended, neutralizing the acidic catalyst by addition of a base such that the resulting mixture has a pH of 8 - 10, then
(g) removing the excess alcohol by distillation at from 0.01 to 10 mbar down to a residual content of less than 5% by weight, based on the amount of alkyl glycoside present, and
(h) bleaching the reaction mixture at pH 8 - 10, after conversion into an aqueous paste having a content of 30 - 70% by weight of alkyl glycoside, using a compound eliminating active oxygen.

2. A process for preparing alkyl glycosides as claimed in claim 1, wherein the aqueous glycose employed is a solution which mainly contains glucose as the glycose.

3. A process for preparing alkyl glycosides as claimed in claim 1 or 2, wherein the alcohols employed are aliphatic primary alcohols having from 8 to 20 C atoms.

4. A process for preparing alkyl glycosides as claimed in one of claims 1 to 3, wherein the acidic catalysts used are alkylbenzenesulfonic acids, alkylsulfonic acids, sulfosuccinic acid mono- and diesters, sulfoalkylcarboxylic acids and -carboxylic acid esters and also mono- and dialkylnaphthalenesulfonic acids.

## Revendications

1. Procédé de préparation d'alkylglycosides par réaction d'oses aqueux ayant une teneur en eau de 10 à 80% en poids avec des alcools primaires aliphatiques à 8-30 atomes de carbone, caractérisé en ce que
a) on met en réaction les alcools et les oses dans un rapport molaire de 2:1 à 10:1,
b) on utilise, comme catalyseur acide ayant des propriétés émulsifiantes, de 0,1 à 5% en poids, par rapport à la quantité des oses mis en réaction, de la forme acide d'un tensio-actif anionique,
c) on utilise, comme autre émulsifiant, de 1 à 30% en poids d'alkylglycoside par rapport à la quantité des oses mis en réaction,
d) on préchauffe les oses aqueux à 50-90°C et on ajoute la solution aqueuse préchauffée des oses, sous mélange efficace, au mélange réactionnel de l'alcool, du catalyseur acide et de l'émulsifiant, de sorte qu'il en résulte une émulsion,
e) on mène la réaction en émulsion à une température de 100 à 150°C et sous une pression de 10 à 100 mbar, l'eau introduite dans le mélange réactionnel et celle qui est formée par la réaction étant chassées de façon continue par distillation,
f) après l'achèvement de la réaction, on neutralise le catalyseur acide par addition d'une base, de sorte que le mélange résultant présente un pH de 8 à 10, puis
g) on chasse par distillation l'alcool en excès, sous une pression de 0,01 a 10 mbar, jusqu'à une teneur résiduelle de moins de 5% en poids, par rapport à la quantité de l'alkylglycoside présent, et
h) après transformation du mélange réactionnel en une pâte aqueuse ayant une teneur de 30 à 70% en poids d'alkylglycoside, on le blanchit à un pH de 8 à 10 avec un composé libérant de l'oxygène actif.

2. Procédé de préparation d'alkylglycosides selon la revendication 1, caractérisé en ce que l'on met en réaction, en tant qu'oses aqueux, une solution qui contient principalement du glucose en tant qu'ose.

3. Procédé de préparation d'alkylglycosides selon la revendication 1 ou 2, caractérisé en ce que l'on met en réaction, en tant qu'alcools, des alcools primaires aliphatiques à 8-20 atomes de carbone.

4. Procédé de préparation d'alkylglycosides selon l'une quelconque des revendications 1 à 3, caractérisé en ce que l'on utilise, comme catalyseurs acides, des acides alkylbenzènesulfoniques, des acides alkylsulfoniques, des mono- et diesters d'acide sulfosuccinique, des acides sulfoalkylcarboxyliques et des esters de ces acides, ainsi que des acides mono- et dialkylnaphtalènesulfoniques.
